Europäisches Patentamt

European Patent Office

Office européen des brevets

(11) Numéro de publication: **0 285 474 B1**

(19)

(12) FASCICULE DE BREVET EUROPEEN

(45) Date de publication de fascicule du brevet: **09.12.92** (51) Int. Cl.⁵: **A61L 27/00**, A61F 2/10

(21) Numéro de dépôt: **88400502.6**

(22) Date de dépôt: **03.03.88**

Le dossier contient des informations techniques présentées postérieurement au dépôt de la demande et ne figurant pas dans le présent fascicule.

(54) **Equivalent de peau.**

(30) Priorité: **26.03.87 FR 8704205**
**19.06.87 FR 8708604**

(43) Date de publication de la demande:
**05.10.88 Bulletin 88/40**

(45) Mention de la délivrance du brevet:
**09.12.92 Bulletin 92/50**

(84) Etats contractants désignés:
**AT BE CH DE ES FR GB GR IT LI NL SE**

(56) Documents cités:
**WO-A-83/01384**
**US-A- 4 485 096**
**US-A- 4 485 097**
**US-A- 4 539 716**
**US-A- 4 604 346**

**Skin Pharmacol 1990, 3:97-106**

**British Journal of Dermatology (1984) 111, Supplement 27, 219-222**

**British Journal of Dermatology (1990) 114, 91-101**

(73) Titulaire: **CENTRE INTERNATIONAL DE RE-CHERCHES DERMATOLOGIOUES GALDERMA - CIRD GALDERMA**
**Sophia Antipolis**
**F-06560 Valbonne(FR)**

(72) Inventeur: **Bernard, Bruno**
**82 Hameaux du Val Bosquet**
**F-06600 Antibes(FR)**
Inventeur: **Lenoir, Marie-Cécile**
**3, rue Dei Suves Le Ht Sartoux**
**F-06560 Valbonne(FR)**
Inventeur: **Shroot, Braham Villa 35**
**Hameaux du Val Bosquet Chemin de Val Bosquet**
**F-06600 Antibes(FR)**
Inventeur: **Darmon, Yves-Michel**
**300 Chemin de la Suquette Vergers de Val Constance**
**F-06600 Antibes(FR)**
Inventeur: **Asselineau, Daniel**
**La Bastide Domaine de Pierrefeu Chemin de la Tour**
**F-06560 Valbonne(FR)**

Rank Xerox (UK) Business Services

Developement Biology 130, 610-620 (1988)

The Journal of Investigative Dermatology, vol. 7, No. 5, November 1986, 642-647

(74) Mandataire: **Michardière, Bernard et al**
**C/O CABINET PEUSCET 68, rue d'Hauteville**
**F-75010 Paris(FR)**

## Description

L'invention se rapporte à un équivalent de peau.

On sait que l'on cherche à obtenir in vitro des structures cellulaires comparables à la peau animale ou humaine, notamment pour pouvoir les utiliser pour des greffes à pratiquer sur des blessés tels que des grands brûlés. On cherche, bien entendu, des structures ayant des similitudes aussi grandes que possible avec la structure de la peau humaine pour écarter les risques de rejet de la greffe. En outre, il est possible d'utiliser un tel matériau pour étudier les effets pharmacologiques ou cosmétiques de divers produits et une telle étude donne des résultats d'autant plus fiables que les ressemblances du matériau avec la peau sont plus fortes.

Dans le brevet US-A-4 485 096, on a décrit la préparation d'un équivalent de peau comprenant un équivalent de derme, sur lequel est ajoutée une suspension de cellules d'épiderme. Ces cellules se plaquent sur l'équivalent de derme pour former une couche avec une certaine différenciation des kératinocytes puisqu'il y a formation d'un stratum cornéum imperméable de cellules granuleuses à kératohyaline et de liaisons desmosomiques. Cependant, l'équivalent d'épiderme ainsi obtenu a une structure qui diffère assez fortement de celle de la peau naturelle ; en particulier, il est indiqué, à cet égard, dans le brevet US-A-4 604 346, que l'adhérence dermique/épidermique n'est pas optimum et que l'équivalent d'épiderme obtenu n'est pas toujours multi-couches et est même parfois incomplet. En outre, on a constaté que ces équivalents de peau ne permettent pas une recolonisation d'un greffon par des mélanocytes.

Dans le brevet US-A-4 604 346 précité, on décrit également la préparation d'un équivalent de peau par utilisation de biopsies d'une peau naturelle ; selon ce procédé, on prélève un morceau d'épiderme naturel lié à son derme, de façon que l'épiderme puisse se développer au niveau de la surface supérieure de l'équivalent de derme. Dans cet équivalent de peau, l'équivalent de derme contient donc des morceaux de derme naturel, de sorte que l'équivalent de peau est nécessairement hétérogène puisque l'explant y reste présent. Si l'explant en était retiré, l'équivalent d'épiderme présenterait des trous, ce qui le rendrait difficilement utilisable pour des greffes. En outre, l'implant de biopsies réalisé entraîne un ensemencement par une population dermique non contrôlée contenant des cellules pouvant induire un rejet d'une greffe réalisée avec cet équivalent de peau.

L'invention se propose donc de décrire un équivalent de peau ayant avec la peau des ressemblances structurales plus importantes que celles présentées par les matériaux de ce type actuellement connus.

La capacité de culture des kératinocytes dans un milieu nutritif approprié est connue. Plus précisément, l'article de F. K. Noser (publié dans "The Journal of Investigative Dermatology", 87, 485-488, 1986) montre la capacité de culture des kératinocytes de la gaine d'un follicule de cheveu humain. En outre, l'article de A.J.M. Vermorken (publié dans "Molec. Biol. Rep.", 10, 205-213, 1985) indique que la culture des kératinocytes de la gaine d'un follicule pileux peut conduire à la formation d'une nappe cellulaire comportant une pluralité de couches qui présentent une certaine différenciation, d'une façon quelque peu analogue à ce que l'on constate dans un épiderme animal ou humain. Il est suggéré que cette fabrication de nappe cellulaire pourrait être effectuée en utilisant comme support un équivalent de derme de type connu mais aucune précision n'est fournie à cet égard. Au surplus, la culture en immersion, qui est décrite, conduit à une structure de nappe cellulaire présentant encore des différences sensibles avec celle d'un épiderme animal ou humain.

La demande internationale PCT publiée sous le numéro WO-A-86/02273 décrit un procédé d'obtention d'un équivalent de peau qui utilise la capacité de culture des kératinocytes et emploie, comme source de kératinocytes, des biopsies de peau. Selon ce procédé on obtient, par extraction acide douce, par exemple à partir de tendons de queue de rat, du collagène de type I que l'on maintient en solution dans un environnement légèrement acide. On cultive, d'autre part, sur un milieu de culture, des fibroblastes obtenus, par exemple, à partir de tissus humains. La solution de collagène est neutralisée par une base et additionnée d'éléments nutritifs. Les fibroblastes y sont ajoutés. On a alors formation d'un gel qui se contracte suite aux interactions entre les fibroblastes et les molécules de collagène, en expulsant le milieu nutrititf pour former un équivalent de derme. Avant que ce gel ne se contracte, on y insère une biopsie de peau de 2 mm de diamètre environ, que l'on positionne de façon que l'épiderme de la biopsie affleure la surface du gel. On observe alors une migration et une multiplication des kératinocytes issus de la biopsie à la surface de l'équivalent de derme ; les phases de différenciation des kératinocytes conduisent finalement à la formation des différentes couches d'un équivalent d'épiderme.

Un tel procédé présente cependant des inconvénients. On peut citer, par exemple, le fait que l'on n'obtient pas un équivalent de peau globalement homogène , puisque la biopsie, y compris sa partie dermique, reste insérée dans l'équivalent de derme. Un autre inconvénient réside dans le mode même de prélèvement de la biopsie, puisqu'elle doit être réalisée par un médecin, qu'elle peut être douloureuse et que des cicatrices visibles peuvent en résulter. De plus, le risque de contamination du manipulateur par des agents infectieux tels que les virus est non négligeable. Enfin, la surface d'équivalent d'épiderme que l'on peut espérer obtenir est limitée par le nombre de biopsies que l'on peut prélever sur un même être humain par exemple.

Le procédé pour l'obtention de l'équivalent de peau selon l'invention utilisé de façon particulière et originale la capacité de culture des kératinocytes contenus dans la gaine d'un follicule pileux. On remplace la biopsie utilisée dans le document WO-A-86/02273 par un follicule pileux entouré de sa gaine et implanté sensiblement perpendiculairement à la surface libre de l'équivalent de derme en cours d'élaboration. On obtient alors des résultats tout à fait surprenants pour l'homme de métier en ce qui concerne la différenciation des kératinocytes ; on obtient en effet un équivalent d'épiderme, dont les ressemblances avec un épiderme humain sont absolument inattendues. Ainsi, bien que les kératinocytes soient, dans le document WO-A-86/02273 comme dans l'invention, la source de la formation d'un équivalent d'épiderme, l'insertion d'un follicule pileux perpendiculairement à la surface libre de l'équivalent de derme en cours d'élaboration, n'est pas équivalente à celle d'une biopsie de la peau quant aux résultats obtenus et améliore ces derniers de façon notable. En outre, la structure de la nappe cellulaire, qui constitue l'équivalent d'épiderme obtenu, présente, vis à vis de la structure de la peau, des ressemblances beaucoup plus importantes que celles de la nappe cellulaire que l'on obtenait en cultivant différemment les mêmes cellules de la gaine d'un follicule pileux (article de A.J.M. Vermorken précité). Enfin la liaison entre les nappes constituées par l'équivalent de derme et l'équivalent d'épiderme est satisfaisante et permet un maniement aisé de l'équivalent de peau.

L'équivalent de peau selon l'invention ainsi constitué est bien différencié, bien organisé. Il est aussi étanche et globalement homogène : en effet, on peut, en cours de procédé, retirer de l'équivalent de derme le (ou les) follicule(s) pileux et le (ou les) trou(s) ainsi formé(s) dans l'équivalent de peau se rebouche(nt).

La différenciation atteinte in vitro dans l'équivalent de peau suivant l'invention est très proche de ce que l'on observe in vivo. On détecte, notamment, la filaggrine dans la couche granuleuse de l'épiderme, comme dans le cas de la peau normale, et l'on peut observer, par électrophorèse, que la filaggrine a bien été obtenue par coupure de la profilaggrine au niveau des ponts oligopeptidiques. D'autre part on retrouve une disposition palissadique des cellules basales qui est notamment la conséquence de l'implantation des follicules pileux perpendiculairement à la surface de l'équivalent de derme en cours d'élaboration.

On obtient en implantant un follicule pileux une surface d'équivalent de peau sensiblement de même importance que celle obtenue avec une biopsie ; mais on peut prélever un grand nombre de ces follicules et obtenir ainsi avec un même donneur une très grande surface, ce qui n'est pas le cas avec des biopsies.

L'équivalent de peau ainsi obtenu, peut être utilisé, notamment, pour le traitement des blessures par greffe de cet équivalent de peau. Dans ce cas, le receveur et le donneur peuvent être des êtres humains et le donneur du follicule pileux peut être le même que le receveur de l'équivalent de peau.

Dans le procédé d'obtention d'un équivalent de peau, selon l'invention :

1°) on prépare un équivalent de derme en mélangeant :

a) des cellules contractiles récoltées, par exemple, à partir de cultures monocouches réalisées sur un milieu nutritif ensemensé par des fragments de tissu animal ou humain

avec

b) un milieu nutritif (MN1) additionné de composants de la matrice extra-cellulaire du derme,

ledit mélange formant un gel qui se contracte en expulsant le milieu nutritif pour former l'équivalent de derme ;

2°) on utilise l'équivalent de derme obtenu selon 1°) comme substrat pour un équivalent d'épiderme obtenu par ensemencement dudit substrat avec les kératinocytes d'un explant animal ou humain et par maintien de conditions permettant la multiplication des kératinocytes à la surface dudit substrat, le développement de cette culture de kératinocytes étant favorisé par l'utilisation d'au moins un milieu (MN1, MN2) au contact desdits kératinocytes, caractérisé par le fait que l'on ensemence le substrat au moyen d'au moins un follicule pileux ou un tronçon de follicule pileux extrait d'une peau humaine ou animale par arrachement, ledit follicule ou tronçon comportant au moins partiellement sa gaine cellulaire et étant implanté dans le substrat de façon que sa ligne longitudinale moyenne soit sensiblement perpendiculaire à la surface libre du substrat.

On peut utiliser comme cellule contractile, des fibroblastes ; on utilise, avantageusement, comme cellules contractiles, des fibroblastes dermiques obtenus à partir de donneurs humains sains et récoltées, à partir des cultures monocouches, par trypsinisation ménagée.

On utilise, de préférence, comme milieu nutritif pour les cultures de cellules contractiles, du milieu essentiel minimum (MEM) ; on utilise avantageusement, comme milieu de culture contenant des composants de la matrice extracellulaire du derme, un milieu contenant au moins du collagène, notamment de

4

type I.

Selon un mode préféré de réalisation, on prépare l'équivalent de derme par les étapes suivantes :

a) on obtient une solution faiblement acide de collagène par extraction acide douce à partir d'une source animale ou humaine ; ce collagène peut aussi être d'origine commerciale, vendu, par exemple, par la société "BIOETICA".

b) on mélange la solution de l'étape a) avec les cellules contractiles et un milieu nutritif (MN1), on neutralise par addition d'une base et on coule le mélange dans un réceptacle plat;

c) on laisse le gel ainsi obtenu se contracter pendant plusieurs jours.

On utilise, de préférence, pour le milieu nutritif (MN1), la formulation suivante :

- de 80 % à 100% en volume d'un milieu essentiel minimum (MEM);
- de 0 % à 20 % en volume de sérum de veau foetal;
- de 0 % à 20 % en volume de sérum humain de group AB ;
- de 0 % à 1 % en poids de produit(s) anti-fongique(s);
- de 0 % à 10 % en poids d'antibiotique(s) ;
- de 0 % à 2 % en poids de composé(s) énergétisants;
- de 0 % à 2 % en poids d'acides aminés non-essentiels.

On peut enfoncer l'implant dans le substrat à partir de la surface libre du substrat d'une distance comprise entre 0,1 et 2 mm. De préférence, on obtient l'implant en coupant le follicule pileux arraché, sensiblement perpendiculairement à sa ligne longitudinale moyenne, dans la zone où il est entouré de sa gaine cellulaire, pour conserver une longueur comprise entre 0,5 et 5 mm environ. Avantageusement, on coupe le follicule pileux au voisinage de sa base pour éliminer le bulbe. De préférence, on insère l'implant dans le substrat dès le début de la contraction du gel, qui constitue le substrat.

Avantageusement, après l'ensemencement du substrat, on maintient pendant un temps $T_1$ le substrat implanté en immersion dans le milieu nutritif (MN1), qui recouvre le (ou les) implant(s), et on peut choisir un temps $T_1$ d'environ cinq à six jours.

Quand le temps $T_1$ est expiré, on remplace avantageusement le milieu (MN1) par un milieu (MN2) que l'on fait affleurer à un niveau situé dans l'épaisseur de l'équivalent de derme. De préférence, pour assurer l'affleurement du milieu (MN2) au niveau désiré, on place l'équivalent de derme sur une grille-support surélevée par rapport au fond du réceptacle et on ajuste le niveau du milieu (MN2), pour que la grille-support soit juste recouverte mais que le milieu (MN2) ne recouvre pas la face supérieure de l'équivalent de peau en cours d'élaboration.

On peut utiliser, comme milieu (MN2), la formulation suivante :

- de 80 % à 100 % en volume de milieu essentiel minimum(MEM);
- de 0 % à 10 % en poids d'antibiotique(s) et/ou anti-fongique(s) ;
- de 0 % à 20 % en volume de sérum animal ou humain ;
- de 0 % à 0,05 % en poids de facteur(s) de croissance.

De préférence, on extrait l'implant du substrat par arrachement à un temps T2 après l'implantation. On peut choisir un temps T2 d'environ 8 à 13 jours et on peut maintenir l'équivalent de peau en contact avec le milieu de culture jusqu'à un temps T3 après l'implantation dans l'équivalent de derme. On choisit avantageusement un temps T3 supérieur à T2 et permettant un recouvrement total du substrat par les kératinocytes développés sur ledit substrat et une différenciation appréciable desdits kératinocytes.

On peut ensemencer le substrat par des implants régulièrement répartis et espacés les uns des autres d'environ 0,5 à 2,5 cm et l'on choisit de préférence un temps T3 d'environ 15 jours à plusieurs mois.

L'invention se rapporte à un équivalent de peau constitué d'un équivalent de derme recouvert d'un équivalent d'épiderme, l'équivalent de derme étant un film formé d'un gel de collagène de type I renfermant des fibroblastes répartis tridimensionnellement dans ledit film, caractérisé par le fait que l'équivalent d'épiderme comporte :

a) un équivalent de membrane basale constitué par un dépôt en couche de laminine, de fibronectine, de collagène de type IV et d'antigène de la pemphigoïde bulleuse ;

b) des cellules de couche basale en disposition palissadique reliées à l'équivalent de membrane selon a) par des hémidesmosomes ;

c) des cellules de couches suprabasales, qui, dès la première couche suprabasale, contiennent de la kératine basique de 67 kDa et de la kératine acide de 56,5 kDa ;

d) des cellules granuleuses qui contiennent des grains de kératohyaline, de l'involucrine, de la transglutaminase et de la filaggrine ;

e) des cellules aplaties et kératinisées disposées au voisinage de la surface libre, donnant, après extraction au dodécyl sulfate de sodium et au 2-mercapto éthanol, des enveloppes cornées, caractéristiques des cornéocytes, les cellules des différentes couches étant reliées entre elles par des desmosomes.

Afin de mieux faire comprendre l'objet de l'invention, on va en décrire ci-après, à titre d'exemple purement illustratif et non limitatif, un mode de mise en oeuvre représenté sur le dessin annexé.

Sur ce dessin :

- la figure 1 est une coupe partielle d'un échantillon de peau (notamment de cuir chevelu) dans lequel est planté un follicule pileux (notamment un cheveu) ;
- la figure 2 représente le cheveu de la figure 1 arraché hors de l'échantillon de cuir chevelu ;
- la figure 3 montre un équivalent de peau en cours d'élaboration, immergé dans un milieu nutritif ;
- la figure 4 montre l'équivalent de peau de la figure 3 mis au contact de l'air dans une phase ultérieure ;
- la figure 5 est une coupe partielle de l'équivalent d'épiderme d'un équivalent de peau selon l'invention.

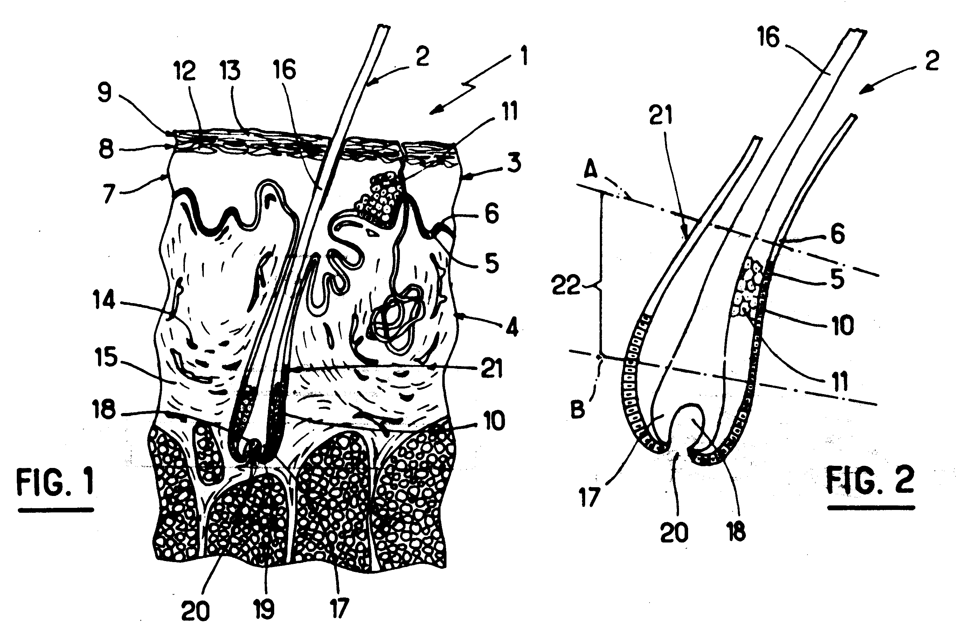

Sur la figure 1 on voit un échantillon de peau, notamment de cuir chevelu, référencé 1 dans son ensemble dans lequel est planté un follicule pileux 2. Le follicule se développe à partir de l'épiderme désigné par 3 dans son ensemble. L'épiderme est séparé du derme désigné par 4 dans son ensemble par une membrane basale 5. On distingue dans l'épiderme quatre niveaux, qui sont en partant de la membrane basale vers l'extérieur de la peau, la couche basale 6, la couche de Malpighi 7, la couche granuleuse 8 et la couche cornée 9.

L'épiderme est constitué à 95 % de cellules nommées kératinocytes. Ces kératinocytes subissent une différenciation depuis la couche basale jusqu'à la couche cornée. La couche basale 6 est constituée d'un seul niveau de kératinocytes 10, de forme sensiblement parallélipipédique ; ces cellules ont un grand axe sensiblement perpendiculaire à la membrane basale 5 et sont reliées à cette dernière par leur base grâce à des hemidesmosomes 39. La couche de Malpighi 7 est constituée essentiellement par un empilement de couches de kératinocytes polyédriques 11. Entre la couche de Malpighi 7 et la couche cornée 9, se situent 2 ou 3 couches de kératinocytes aplatis 12 constituant la couche granuleuse 8. Enfin, la couche cornée 9 se compose d'un empilement de cellules sensiblement hexagonales 13, mortes, aplaties et bien ordonnées.

Le derme 4 comporte des fibroblastes 14, noyés dans une matrice extracellulaire 15 composée de macromolécules telles que des collagènes, des mucopolysaccarides, des glycoaminoglycanes ou de la fibronectine.

le cheveu 2 se compose d'un corps 16, dont la partie plantée dans l'épiderme se termine par un bulbe 17. Le bulbe 17 présente, à sa base, une cavité 18 qui contient des papilles dermiques 19, et qui s'ouvre sur le derme par un orifice 20. Une partie de la membrane basale 5 de l'épiderme enveloppe partiellement le corps 16 du cheveu 2, ainsi que le bulbe 17 et se lie à ce dernier en laissant ouvert l'orifice 20. Cette partie de la membrane basale constitue ainsi une gaine 21 délimitée extérieurement par des kératinocytes 10 de la couche basale et renfermant entre cette couche et le cheveu, des kératinocytes polyédriques 11 de la couche de Malpighi.

Une des étapes du procédé consiste à préparer un implant constitué d'une partie du cheveu 2. Cette préparation s'effectue comme suit : le cheveu est prélevé facilement sans douleur et sans risque d'infection à l'aide d'une pince par exemple. Cette opération ne nécessite aucune qualification médicale ; un tel cheveu arraché est représenté sur la figure 2.

Lors de cette extraction de l'implant, la gaine 21 se sépare de la couche basale environnante par rupture de ladite couche. Deux cas peuvent alors se produire : ou bien, lors de l'extraction, la couche basale se rompt au niveau du bulbe et l'on extrait alors uniquement le corps 16 du cheveu, la gaine 21 restant dans l'épiderme 3 ; ou bien la membrane basale se rompt au-dessus du bulbe, notamment au niveau de la jonction derme/épiderme, et l'on obtient alors un cheveu, dont la partie inférieure est enveloppée dans la gaine 21. Seule cette deuxième éventualité est retenue dans l'étape de préparation de l'implant ; les cheveux ou les poils arrachés sans gaine ne sont pas utilisés. Si les papilles dermiques sont restées dans l'épiderme 3 lors de l'extraction, le cheveu 2, une fois prélevé, ne contient ni fibroblaste ni contaminant dermique quelconque, ces derniers étant en fait des éléments gênants dans une culture . Par ailleurs, les papilles dermiques pourront éventuellement redonner naissance à un cheveu et recréer une gaine 21 contenant des kératinocytes 10 et 11. Le follicule pileux représente donc une source pratiquement infinie de kératinocytes.

On coupe ensuite le cheveu arraché, sensiblement perpendiculairement à sa ligne longitudinale moyenne, dans la zone où il est entouré de sa gaine 21, aux endroits A et B repérés sur la figure 2, tels que B se trouve au-dessus du bulbe et A au-dessus de B, pour conserver une longueur AB comprise entre 0,5 et 5 mm environ.

On élimine ainsi le bulbe 17 car, d'une part, l'extrémité molle pourrait être gênante dans la suite du procédé comme explicité ci-après, et, d'autre part, on élimine ainsi tout risque de contamination par des papilles dermiques qui seraient restées au niveau du bulbe. On obtient alors un implant 22 prêt à être utilisé.

Parallèlement à cette préparation de l'implant 22, une autre étape du procédé consiste en la préparation d'une structure équivalente au derme 4 que l'on nommera dans la suite un équivalent de derme. Cette préparation se réalise, de façon connue, comme indiqué ci-après.

On prélève sur des donneurs humains des fragments de tissu cutané comprenant du derme et de l'épiderme. Ces fragments sont disposés dans une boîte dans un milieu de culture qui est du "Milieu Essentiel Minimum" (MEM) vendu, par exemple, par la Société SEROMED, et dont une composition classique est donnée dans le tableau I ci-après.

TABLEAU I

FORMULATION DU MEM

| Composants | Quantités en mg/l | Composants | Quantités en mg/l |
|---|---|---|---|
| NaCl | 6800 | L-Lysine.HCl | 73 |
| KCl | 400 | L-Méthionine | 15 |
| Na$_2$HPO$_4$.2H$_2$O | - | L-Phénylalanine | 32 |
| NaH$_2$PO$_4$.H$_2$O | 140 | L-Thréonine | 48 |
| KH$_2$PO$_4$ | - | L-Tryptophane | 10 |
| MgSO$_4$.7H$_2$O | 200 | L-Tyrosine | 36 |
| CaCl$_2$ | 200 | L-Valine | 46 |
| D-Glucose | 1000 | D-Ca-Pantothénate | 1 |
| Phénol rouge | 10 | Pyridoxal.HCl | 1 |
| NaHCO$_3$ | 2200 | Thiamine.HCl | 1 |
| L-Arginine.HCl | 126 | Riboflavine | 0,1 |
| L-Cystine | 24 | i-Inositol | 2 |
| L-Glutamine | 292 | Acide folique | 1 |
| L-Histidine.HCl.H$_2$O | 42 | Chlorure de choline | 1 |
| L-Isoleucine | 5,2 | Nicotinamide | 1 |
| L-Leucine | 52 | | |

Les fibroblastes du derme quittent alors le tissu cutané et se multiplient en monocouche sur le fond de la boîte.

On prépare, d'autre part, une solution faiblement acide de collagène de type I, obtenue par extraction acide douce à partir de tendons de queue de rat.

On mélange ensuite cette solution faiblement acide de collagène avec des fibroblastes, cultivés en monocouche comme indiqué précédemment et récoltés par trypsinisation ménagée à partir de cultures subconfluentes ou confluentes, en présence d'un milieu nutritif (MN1), ayant la composition suivante :

- 85à90% en volume de MEM ;
- 10% en volume de sérum de veau foetal ou 10 % en volume de sérum humain AB ;
- 1 % en poids d'acides aminés non-essentiels (indiqués dans le tableau II ci-après).

## TABLEAU II
### ACIDES AMINES NON-ESSENTIELS

| Composants | Quantités en mg/l |
|---|---|
| L-Alanine | 8,90 |
| L-Asparagine.$H_2O$ | 15,00 |
| Acide L-Aspartique | 13,30 |
| Acide L-Glutamique | 14,70 |
| L-Glycine | 7,50 |
| L-Proline | 11,50 |
| L-Sérine | 10,50 |

- 100 U/ml de pénicilline
- 100 μg/ml de streptomycine
- 2,5 μg/ml d'amphotéricine B
- 1 mM de pyruvate de sodium

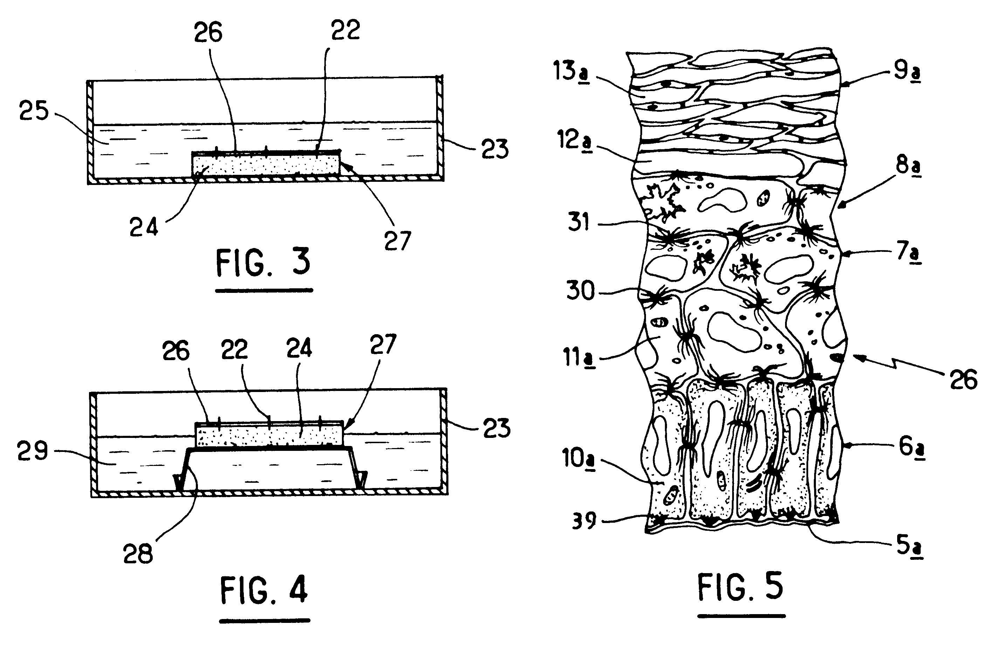

On neutralise le mélange avec de la soude 0,1 N et on le coule dans un réceptacle plat 23 comme représenté sur la figure 3. Le mélange prend en gel très rapidement.

Suite aux interactions entre les fibroblastes, qui sont des cellules contractiles, et les fibres de collagène contenues dans le milieu nutritif gélifié, le volume du gel diminue, le milieu nutritif est expulsé et un équivalent de derme 24 se forme. On laisse cette contraction du gel s'effectuer pendant quelques jours ; le volume du gel se réduit d'au moins vingt fois.

Les fibroblastes ne constituent pas les seules cellules contractiles utilisables. On pourrait aussi utiliser, par exemple, les cellules des muscles lisses, les cellules des muscles striés ainsi que celles du muscle cardiaque ; on peut aussi citer les plaquettes sanguines.

De même, si l'on a utilisé, dans cet exemple, une solution de collagène de type I comme composant de la matrice extracellulaire de derme, il existe cependant plusieurs collagènes, qui conviennent pour la reconstitution d'un équivalent de derme ; ces collagènes doivent, de préférence, être faits de molécules complètes possédant encore leurs télopeptides. Le collagène de type I fait partie de ce groupe mais on peut citer également le collagène humain extrait du placenta après digestion enzymatique partielle.

La préparation du gel, qui forme l'équivalent de derme 24, étant achevée, on entame alors l'étape suivante du procédé, qui est l'épidermisation de cet équivalent de derme.

L'implant 22, obtenu comme indiqué précédemment, est inséré dans le gel pendant les premières minutes de contraction, de façon que sa ligne longitudinale moyenne soit sensiblement perpendiculaire à la surface libre du gel. C'est dans cette phase d'insertion de l'implant 22 que le bulbe du follicule pileux peut être gênant car sa partie molle ne facilite pas l'insertion. C'est pourquoi il est préférable de couper le bulbe dans la préparation de l'implant.

Durant la phase de contraction du gel, qui va donner naissance à l'équivalent de derme 24, l'implant 22 se retrouve solidement tenu en place dans le gel. On a représenté sur la figure 3 un équivalent de derme 24 dans lequel sont insérés plusieurs implants 22. En effet il est possible d'ensemencer l'équivalent de derme en cours d'élaboration par des implants régulièrement répartis et espacés par exemple les uns des autres d'environ 0,5 à 2,5 cm. Après cet ensemencement, on maintient pendant 5 à 6 jours l'équivalent de derme 24 en immersion dans le milieu nutritif 25 (MN1). Ce milieu de culture 25 recouvre les implants comme représenté sur la figure 3. Pendant cette phase, la multiplication des kératinocytes des gaines 21 des implants débute, et on assiste alors, à partir des implants, à un mouvement radial et vertical des kératinocytes à la surface de l'équivalent de derme 24.

Cette migration des kératinocytes va conduire, au terme de l'épidermisation, à la formation sur l'équivalent de derme d'un équivalent d'épiderme 26.

L'ensemble (équivalent de derme 24/équivalent d'épiderme 26) va donc constituer un équivalent de peau 27.

A la fin de cette période de 5 à 6 jours, on remplace le milieu (MN1) par un milieu 29 (MN2) ayant la formulation suivante :

- 85 à 90% en volume de Milieu Essentiel Minimum (MEM)
- 0 à 10% en poids d'antibiotiques (penicilline, streptomycine, amphotéricine B)
- 10 % en volume de sérum animal ou humain
- 0à0,05% en poids de facteurs de croissance (facteur de croissance épidermique, hydrocortisone, toxine cholérique).

Dans le même temps, l'équivalent de peau 27 est disposé sur une grille d'acier inoxydable 28 reposant sur le fond du réceptacle 23. La grille maintient l'équivalent de peau surélevé dans la boîte de culture 23, au voisinage de l'interface air-liquide, tel que représenté sur la figure 4. Le niveau du milieu MN2 est ajusté de telle sorte que la grille-support 28 soit juste recouverte mais que le milieu MN 2 ne recouvre pas la face supérieure de l'équivalent de peau 27 en cours d'élaboration. Le fait de placer ainsi l'équivalent de peau en cours d'élaboration au contact de l'air et d'assurer l'alimentation des kératinocytes par l'équivalent de derme, favorise la différenciation des kératinocytes et donc la formation d'un équivalent d'épiderme bien structuré .

Après 8 à 13 jours, les implants sont retirés tandis que l'équivalent d'épiderme en formation reste sur l'équivalent de derme. Le diamètre des implants étant très faible, de l'ordre de 0,5 mm environ, les trous résultant de l'extraction des implants hors de l'équivalent de peau se rebouchent et on obtient donc un équivalent de peau globalement homogène, sans trou, et étanche.

Une fois les implants 22 retirés, on laisse l'équivalent de peau 27 en cours d'élaboration au contact du milieu MN2 pendant un temps permettant un recouvrement total de l'équivalent de derme par les kératinocytes développés sur ce dernier. La croissance de l'équivalent d'épiderme est mesurée en colorant la couverture épithéliale au bleu du Nil en solution aqueuse à 1/10000 et en mesurant la surface de l'équivalent d'épiderme.

La surface d'un équivalent de peau 27 varie en fonction du nombre d'implants insérés dans l'équivalent de derme et en fonction de la surface de celui-ci. Ainsi, avec un seul implant, d'un diamètre d'environ 0,5 mm, on obtient un équivalent de peau d'une surface d'environ 1 cm$^2$ au bout de 10 à 12 jours . On peut bien entendu obtenir une surface beaucoup plus grande en ensemençant l'équivalent de derme par des implants régulièrement répartis et espacés les uns des autres d'environ 0,5 à 2,5 cm par exemple.

Lorsque cette phase d'épidermisation est terminée, on obtient l'équivalent de peau 27, dont on a représenté sur la figure 5 une coupe partielle de l'équivalent d'épiderme 26. Classiquement, cette coupe a été faite après coloration à l'hémalun - phloxine - safran.

L'équivalent de peau 27 obtenu suivant le mode de réalisation décrit ci-dessus, est donc constitué d'un équivalent de derme recouvert d'un équivalent d'épiderme 26, l'équivalent de derme étant un film formé d'un gel de collagène de type I renfermant des fibroblastes répartis tridimensionnellement dans le film. L'équivalent d'épiderme 26, représenté sur la figure 5, comporte un équivalent de membrane basale 5a, constitué par un dépôt en couche de laminine, de fibronectine, de collagène de type IV et d'antigène de la pemphigoïde bulleuse. Au-dessus de cet équivalent de membrane basale, on trouve des kératinocytes 10a en disposition palissadique reliés à l'équivalent de membrane par des hémidesmosomes 39 : on retrouve donc un équivalent de couche basale 6a. On observe également des cellules progressivement différenciées depuis les cellules 10a de la couche basale 6a jusqu'à des cellules aplaties 13a disposées au voisinage de la surface libre, les cellules des différentes couches étant reliées entre elles par des desmosomes 30. L'équivalent de couche cornée 9a ainsi que les équivalents des couches de Malpighi 7a et granuleuse 8a, sont très semblables aux couches réelles de la peau humaine.

On trouve, d'autre part, dans les kératinocytes de l'équivalent d'épiderme 26, des fibres de kératine 31 et l'on constate que la kératine basique de 67 kDa, qui est un marqueur de différenciation terminale normalement absent dans les cultures de l'état de la technique et dans les follicules de cheveu fraîchement prélevés, est synthétisée de façon normale dans les couches suprabasales 7a et 8a de même que son partenaire, la kératine acide de 56,5 kDa. De plus, cette kératine de 67 kDa est détectée dès la première couche suprabasale 7a comme dans une peau normale.

La filaggrine est aussi présente dans l'équivalent d'épiderme 26 ; on la détecte dans l'équivalent de couche granuleuse 8a ; elle résulte de la maturation protéolytique de son précurseur, la profilaggrine. Par électrophorèse par exemple, on peut voir que cette filaggrine est convenablement coupée au niveau des ponts oligopeptidiques de la profilaggrine. Le procédé selon l'invention, permet donc la maturation de la profilaggrine dans des kératinocytes humains.

L'équivalent d'épiderme 26 ainsi reconstitué est donc bien différencié et bien organisé. Il est également uniforme du point de vue de l'épaisseur et de l'état de différenciation.

Enfin, l'adhérence équivalent de derme/équivalent d'épiderme est excellente. Elle permet donc une manipulation aisée de l'équivalent de peau 27 ainsi formé.

**Revendications**

1. Equivalent de peau (27) constitué d'un équivalent de derme (24) recouvert d'un équivalent d'épiderme (26), l'équivalent de derme étant un film formé d'un gel de collagène de type I renfermant des fibroblastes (14) répartis tridimensionnellement dans ledit film, caractérisé par le fait que l'équivalent d'épiderme (26) comporte :

a) un équivalent de membrane basale (5a) constitué par un dépôt en couche de laminine, de fibronectine, de collagène de type IV et d'antigène de la pemphigoïde bulleuse ;

b) des cellules (10a) de couche basale (6a) en disposition palissadique, reliées à l'équivalent de membrane (5a) selon a) par des hémidesmosomes (39) ;

c) des cellules (11a ; 12a) de couches suprabasales (7a ; 8a), qui, dès la première couche suprabasale (7a), contiennent de la kératine basique de 67 kDa et de la kératine acide de 56,5 kDa ;

d) des cellules granuleuses qui contiennent des grains de kératohyaline, de l'involucrine, de la transglutaminase et de la filaggrine ;

e) des cellules aplaties et kératinisées disposées au voisinage de la surface libre, donnant, après extraction au dodécyl sulfate de sodium et au 2-mercapto éthanol, des enveloppes cornées, caractéristiques des cornéocytes, les cellules des différentes couches étant reliées entre elles par des desmosomes.

**Claims**

1. Skin equivalent (27) composed of a dermis equivalent (24) covered with an epidermis equivalent (26), the dermis equivalent being a film formed from a type I collagen gel containing fibroblasts (14) arranged three-dimensionally in the said film, characterised by the fact that the epidermis equivalent (26) comprises:

a) a basal membrane equivalent (5a) formed from a layer of laminin, fibronectin, type IV collagen and the bullous pemphigus antigen;

b) cells (10a) of basal layer (6a) in a palisade arrangement, attached to the membrane equivalent (5a) according to a) by hemidesmosomes (39);

c) cells (11a; 12a) of suprabasal layers (7a; 8a) which, beginning with the first suprabasal layer (7a), contain basic keratin of 67 kDa, and acidic keratin of 56.5 kDa;

d) granular cells containing grains of keratohyalin, involucrine, transglutaminase and filaggrine;

e) flattened and keratinised calls arranged near the free surface, giving, after extraction using dodecyl sodium sulphate and 2-mercaptoethanol, corneous envelopes characteristic of corneocytes, the cells of the different layers being attached to one another by desmosomes.

**Patentansprüche**

1. Hautäquivalent (27) bestehend aus einem Dermisäquivalent (24), überdeckt von einem Epidermisäquivalent (26),
wobei das Dermisäquivalent einen Film darstellt, welcher durch ein Typ-I-Kollagen-Gel gebildet wird und im Film dreidimensional verteilt Fibroblasten (14) enthält,
**dadurch gekennzeichnet,** daß
das Epidermisäquivalent (26) folgendes umfaßt:

a) ein Basalmembranäquivalent (5a) bestehend aus einer Laminin-Depotschicht, Fibronektin, Typ-IV-Kollagen und dem Antigen des blasigen Pemphigus;

b) Zellen (10a) der Basalschicht (6a) in palissadenförmiger Anordnung, welche mit dem Membranäquivalent (5a) gemäß Punkt a) über Hemidesmosome (39) verbunden sind;

c) Zellen (11a, 12a) der Suprabasalschichten (7a; 8a), die von der ersten suprabasalen Schicht (7a) an basisches Keratin mit 67 kDa und saures Keratin mit 56,5 kDa enthalten;

d) Granuläre Zellen, die Keratohyalin-Körner, Involukrin, Transglutaminase und Filaggrin enthalten;

e) glatte und keratinisierte Zellen im Bereich der freien Oberfläche,

wodurch sich nach Extraktion mit Natriumdodecylsulfat und 2-Mercaptoethanol für Korneozyten charakteristische Hornschichten ergeben, wobei die Zellen der unterschiedlichen Schichten untereinander durch Desmosome verbunden sind.

FIG. 1

FIG. 2

FIG. 5

FIG. 3

FIG. 4